# EUROPÄISCHE PATENTSCHRIFT

(11) **EP 0 510 382 B1**
(45) Veröffentlichungstag und Bekanntmachung des Hinweises auf die Patenterteilung: **18.01.1995**
(21) Anmeldenummer: 92105393.0
(22) Anmeldetag: 28.03.1992
(51) Int. Cl.: C07C 213/02

(54) **Verfahren zur Herstellung von Alpha-Omega-Aminoalkoholen**
Method of preparation of alpha-omega-aminoalcohols
Procédé pour la préparation de alpha-omega-aminoalcools

(30) Priorität: 23.04.1991 DE 4113161
(43) Veröffentlichungstag der Anmeldung: 28.10.1992
(73) Patentinhaber: BASF Aktiengesellschaft, 67063 Ludwigshafen (DE)
(72) Erfinder: Koppenhoefer, Gerhard, W-6725 Roemerberg (DE); Schroeder, Wolfgang, Dr., W-6702 Duerkheim (DE); Voges, Dieter, Dr., W-6800 Mannheim 1 (DE)

(56) Entgegenhaltungen:
- EP-A- 0 021 073
- EP-A- 0 036 331
- EP-A- 0 300 323
- US-A- 3 560 550
- US-A- 4 151 204

## Beschreibung

Die vorliegende Erfindung betrifft ein neues und verbessertes Verfahren zur Herstellung von α,ω-Aminoalkoholen durch Umsetzung von α,ω-Alkandiolen und Ammoniak bei erhöhten Temperaturen und erhöhten Drücken in Gegenwart eines Katalysators, dessen katalytisch aktive Masse zu 5 bis 100 Gew.-% aus Eisen besteht.

α,ω-Aminoalkohole sind vielfältig verwendbare Zwischenprodukte. Ihre Vielseitigkeit resultiert daraus, daß das Molekül zwei verschiedene reaktionsfähige endständige Gruppierungen enthält. Ein bisher bestehender Mangel ist, daß nur der einfachste Vertreter dieser Produktklasse (Ethanolamin) auf einfache Weise synthetisiert werden kann, und zwar durch Ethoxylieren von Ammoniak.

Bisher fehlte ein Weg, um längerkettige, unverzweigte, Aminoalkohole mit endständigen funktionellen Gruppen mit befriedigender Wirtschaftlichkeit herzustellen.

In der EP-A-21 073 wird die Verwendung von Katalysatoren auf der Basis von Eisen und Kobalt zur Herstellung von Aminen aus Alkoholen oder Carbonylverbindungen beschrieben. Aufgrund der Beispiele und der allgemeinen Beschreibung der Katalysatoranwendung handelt es sich hier um den Austausch einer OH-Gruppe in einem Molekül, das außer dieser einen OH-Gruppe leicht abspaltbare andersartige Substituenten enthält, z.B. eine peralkylierte Aminogruppe. Die Anwesenheit von Wasserstoff bei der Reaktion ist nicht erforderlich.

Aus der US-A-4 151 204 ist die Herstellung von Aminoalkoholen unter Verwendung von Eisen- und Kobalt-haltigen Katalysatoren bekannt, jedoch ist hier der Austausch einer leicht austauschbaren sekundären OH-Gruppe gegen eine NH₂-Gruppe beschrieben, so daß Verbindungen erhalten werden, die neben einer primären OH-Gruppe eine sekundäre NH₂-Gruppe im Molekül enthalten.

Aus der US-A-3 652 545 ist bekannt, daß man bei der Umsetzung von 1,6-Hexandiol mit Ammoniak an Raney-Nickel oder Raney-Kobalt oder einem Nickel-auf-Kieselgur-Katalysator zwecks Herstellung von N-(6-aminohexyl)-hexamethylenimin als Nebenprodukt unter anderem 6-Aminohexanol findet. Zu dessen gezielter Herstellung ist dieses Verfahren offensichtlich weder gedacht noch geeignet.

Aus der US-A-3 560 550 ist bekannt, daß man gezielt zum 6-Aminohexanol und verwandten Verbindungen gelangen kann, wenn man ein Lacton zusammen mit Ammoniak bei 250 bis 300°C gasförmig über aktives γ-Al₂O₃ leitet und das entstehende ω-Hydroxinitril an Raney-Nickel zum Aminoalkohol hydriert. Es handelt sich hier also um einen vielstufigen, d.h. aufwendigen und darum unwirtschaftlichen Prozeß.

Aus der GB-A-1 185 310 ist bekannt, daß man 6-Aminohexanol erhalten kann, wenn man ε-Caprolactam zusammen mit Ammoniak bei 220 bis 350°C in einem Autoklav an einem Rutheniumkatalysator mehrere Stunden lang reagieren läßt. Aminohexanol entsteht als Nebenprodukt. Ein wirtschaftliches Verfahren zur Herstellung dieser Verbindung ist daraus nicht zu gestalten.

Der Nachteil dieser Wege besteht u.a. in ihrer Kompliziertheit, in dem Einsatz aufwendig herstellbarer Vorprodukte und in der meistens recht niedrigen Ausbeute.

Der vorliegenden Erfindung lag daher die Aufgabe zugrunde, den zuvor genannten Nachteilen abzuhelfen.

Demgemäß wurde ein neues und verbessertes Verfahren zur Herstellung von α,ω-Aminoalkoholen der allgemeinen Formel I

HO-CH₂-X-CH₂-NH₂ (I),

in der
- X: eine gegebenenfalls durch inerte Reste substituierte und/oder gegebenenfalls durch Sauerstoff oder Stickstoff unterbrochene C₁- bis C₂₀-Alkylenkette
bedeutet, durch Umsetzung von α,ω-Alkandiolen der allgemeinen Formel II

HO-CH₂-X-CH₂-OH (II),

in der das Zwischenglied X die obengenannten Bedeutungen hat, mit Ammoniak und einem Katalysator bei Temperaturen von 150 bis 300°C und Drücken von 50 bis 300 bar, gefunden, welches dadurch gekennzeichnet ist, daß man Katalysatoren einsetzt, deren katalytisch aktive Masse zu 5 bis 100 Gew.-% aus Eisen besteht.

Das erfindungsgemäße Verfahren läßt sich wie folgt durchführen:
Die α,ω-Alkandiole II, gegebenenfalls in einem inerten Lösungsmittel, und Ammoniak werden in Gegenwart eines Katalysators bei einem Druck zwischen 50 und 300 bar, besonders 150 bis 250 bar und Temperaturen zwischen 150 und 300°C, besonders 180 und 270°C, in einer Menge von 0,1 bis 2 l α,ω-Alkandiol II pro l Katalysator und pro Stunde, besonders 0,2 bis 1 l pro l und Stunde zugeführt. Ammoniak und das α,ω-Alkandiol II werden im Molverhältnis von 1:1 bis 300:1, bevorzugt von 1,5:1 bis 100:1, besonders bevorzugt von 2:1 bis 20:1 eingesetzt. Das Ammoniak kann gasförmig oder bevorzugt flüssig eingesetzt werden.

Die Reaktion wird z.B. so gelenkt, daß etwa 50 bis 70 % des α,ω-Alkandiols umgesetzt werden. Der Reaktoraustrag wird gekühlt, z.B. partiell entspannt, das Ammoniak abgetrennt, gegebenenfalls wieder verflüssigt und kann erneut zur Reaktion gebracht werden. Das Reaktionsgemisch kann destillativ zerlegt werden. Die α,ω-Aminoalkohole I erhält man z.B. in Reinheiten von über 98 %, das nicht umgesetzte Diol II kann erneut zur Reaktion gebracht werden. Gegebenenfalls fallen Nebenprodukte an, die ebenfalls Wertprodukte sind, wie z.B. die cyclischen Imine (Morpholin, Pyrrolidin, Piperidin), oder die Diamine (Hexamethylendiamin).

Die Lenkung der Reaktion in den gewünschten Umsatzbereichen erfolgt bevorzugt mit Hilfe der Reaktionstemperatur. Allgemein gilt, daß mit Absenken der Temperatur der Diol-Umsatz zurückgeht, aber die Selektivität in Bezug auf den Aminoalkohol zunimmt. Diese liegt im allgemeinen zwischen 60 und 80 %. Höhere Selektivitäten als 80 % sind zwar möglich, jedoch leidet die Wirtschaftlichkeit wegen des geringeren Umsatzes. Mit einer Steigerung der Temperatur läßt sich der Umsatz des Diols erhöhen, jedoch leidet die Wirtschaftlichkeit wegen der geringeren Selektivität, weil ein steigender Anteil des Diols zu den im Prinzip verwertbaren, aber eigentlich unerwünschten Nebenprodukten reagiert.

Die α,ω-Alkandiole II können entweder in reiner Form oder als Lösung dem Reaktor zugeführt werden. Bevorzugt wird ohne zusätzliches Lösungsmittel gearbeitet, jedoch kann es gelegentlich zweckmäßig sein, eine Lösung einzusetzen. Dies gilt besonders dann, wenn das Diol II einen hohen Schmelzpunkt hat. Als Lösungsmittel eignen sich z.B. cyclische Ether, wie z.B. Tetrahydrofuran, besonders tertiäre Amine, wie z.B. N-Methylmorpholin.

Als Reaktor eignen sich bevorzugt Festbettreaktoren, die von der Reaktionsmischung entweder von unten nach oben oder besser von oben nach unten durchströmt werden. Der Katalysator kann sich darin in Form einer regellosen Schüttung befinden und besteht aus Partikeln beliebiger Gestalt.

Bevorzugt werden Tabletten mit den Hauptmaßen zwischen 4 und 8 mm, oder Strangpreßlinge ähnlichen Formats. Für das erfindungsgemäße Verfahren eignen sich Katalysatoren, deren katalytisch aktive Masse aus 5 bis 100 % Eisen, bevorzugt aus 5 bis 60 Gew.-% Eisen und 0 bis 95 Gew.-% Kobalt bestehen.

Die Katalysatoren enthalten vorteilhaft etwa gleiche Gewichtsmengen an Eisen und Kobalt; ein Gehalt von mindestens 25 % an Kobalt soll in der Regel nicht unterschritten werden. Weniger als 5 % Eisen zuzusetzen, ist in der Regel ebenfalls nicht von Vorteil, weil sich solche Katalysatoren nicht wesentlich von reinen Kobalt-Katalysatoren unterscheiden. Kobalt-Katalysatoren, in denen etwa 25 bis 50 % des Kobalts durch Eisen ersetzt sind, sind in jeder Hinsicht in der Wirkung besonders günstig.

Ein geeignetes Verfahren, um Eisen-Kobalt-Katalysatoren zu erhalten, ist die gemeinsame Fällung aus Lösungen von Eisen- und Kobaltsalzen als Hydroxide bzw. Oxidhydrate bzw. basische Carbonate und Behandlung der gegebenenfalls vorher erhitzten Fällung mit reduzierenden Mitteln. Die Fällung sollte aus verdünnter bis mäßig konzentrierter Lösung geschehen, um einheitliche Fällungen zu erhalten. Etwa 0,1 bis 1 molare Lösungen der Nitrate von Eisen (III) und Kobalt (II) sind besonders geeignet. Auch die Sulfate und die Chloride können verwendet werden.

Man fällt mit Soda- bzw. Pottaschelösung, die z.B. etwa 5- bis 30 %ig sein kann, bei etwa 60 bis 90°C, wobei sich die Fällung über etwa 1 bis 5 Stunden, je nach Art der benutzten Vorrichtung, erstrecken sollte. Man stellt einen pH-Wert um 7 ein, wobei die Fällung bei absatzweisem Verfahren in der Weise erfolgt, daß die Metallsalzlösung in überschüssige Alkalicarbonat-Lösung eingerührt wird, so daß bis zum Ende der Fällung der Niederschlag stets von einem überschuß an Carbonat-Ionen umgeben ist, und schließlich ein pH-Wert von nicht unter 7 erreicht wird. Bei fortlaufender Herstellung können beide Lösungen auch gemeinsam einem Rührgefäß zugeführt werden, wobei ihre Menge so bemessen werden, daß die Mischung einen pH-Wert von 7 oder darüber aufweist, d.h. man legt anfangs einen überschuß an Alkalicarbonat vor und hält diesen aufrecht.

Die vorstehenden Maßnahmen sind zur Erzielung eines guten, insbesondere druckfesten (also mechanisch haltbaren) und trotzdem voluminösen (also porösen und daher hochaktiven) Katalysators entscheidend; an sich ist nämlich bekannt, Eisen-Kobaltkatalysatoren herzustellen, indem man deren Salzlösungen mit z.B. Pottaschelösung fällt (vgl. J. Am. Chem. Soc., 76, S. 319-323 (1954)). Es ist auch bekannt, Eisen-Kobalt-Katalysatoren, die auf verschiedene Weise hergestellt sein können, für die hydrierende Aminierung, z.B. die Herstellung von Hexamethylendiamin oder Ethylendiamin zu verwenden. Die beschriebenen Herstellverfahren (vgl. GB-PS 1 206 659, FR-PS 2 180 462 und US-PS 3 840 479) führen jedoch praktisch stets zu Katalysatoren, die zu wenig porös sind, so daß sie für die feste Anordnung, z.B. in einem Rohrreaktor nur zusammen mit einem Träger verwendet werden können (GB-PS 1 206 659) bzw. in unwirtschaftlich großer Gewichtsmenge verwendet werden müssen.

Der Niederschlag wird wie üblich gewaschen und getrocknet, z.B. bei 100 bis 150°C. Er erleidet beim Glühen einen Verlust von etwa 22 bis 25 % und enthält i.a. etwa 0,25 bis 0,5 % Alkali (Natrium; als Na₂O). Röntgenographisch handelt es sich um ein Gemisch von Kobaltcarbonat (CoCO₃) und - überwiegend - einer Verbindung beider Metalle mit der Struktur des natürlichen Mannasseits; ähnliche künstlich herstellbare Verbindungen sind z.B. in der DE-PS 20 24 282 beschrieben. Verbindungen, die ausschließlich bzw. im wesentlichen Kobalt und Eisen enthalten, sind dort allerdings nicht genannt.

Der Anteil der definierten Verbindung am Gemisch mit Kobaltcarbonat hängt wahrscheinlich mit dem Verhältnis der beteiligten Metalle zusammen.

Die beim Erhitzen auf 300°C oder mehr erhaltene feinteilige Masse ist praktisch röntgenamorph. Sie kann mit etwas Flüssigkeit (Wasser o.ä.) angeteigt, verknetet und zu Formkörpern verarbeitet werden, wie sie bei Katalysatoren üblich sind, z.B. Strängen oder Pellets. Eine Verwendung des unverarbeiteten Pulvers als suspendierter Katalysator ist gleichfalls möglich. Man reduziert wie üblich vor dem Gebrauch mit Wasserstoff. Ebenso ist es möglich, Fällung und Verarbeitung in Gegenwart eines chemisch indifferenten Trägers vorzunehmen, so daß ein Kobalt-Eisenkatalysator entsteht, der z.B. eine besonders große spezifische Oberfläche aufweist.

Das Zwischenglied X in den Verbindungen I und II hat folgende Bedeutungen:
- X: - eine C₁- bis C₂₀-Alkylenkette wie -(CH₂)-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₁₂-, -(CH₂)₁₃-, -(CH₂)₁₄-, -(CH₂)₁₅-, -(CH₂)₁₆-, (CH₂)₁₇-, -(CH₂)₁₈-, -(CH₂)₁₉- und -(CH₂)₂₀-, bevorzugt (CH₂)₂ bis (CH₂)₁₀, besonders bevorzugt (CH₂)₃ und (CH₂)₄.
- eine durch Sauerstoff oder Stickstoff unterbrochene C₁- bis C₂₀-Alkylenkette wie CH₂-O-CH₂ oder 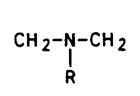 wobei R für Wasserstoff oder Methyl (CH₃) steht.
- eine durch inerte Reste substituierte C₁- bis C₂₀-Alkylenkette, bevorzugt eine durch ein bis fünf inerte Reste substituierte C₁- bis C₂₀-Alkylenkette, besonders bevorzugt eine durch ein bis drei inerte Reste substituierte C₁- bis C₂₀-Alkylenkette,
- eine durch inerte Reste substituierte und durch Sauerstoff oder Stickstoff unterbrochene C₁- bis C₂₀-Alkylenkette, bevorzugt eine durch ein bis fünf inerte Reste substituierte und durch Sauerstoff oder Stickstoff unterbrochene C₁- bis C₂₀-Alkylenkette, besonders bevorzugt eine durch ein bis drei inerte Reste substituierte und durch Sauerstoff oder Stickstoff unterbrochene C₁- bis C₂₀-Alkylenkette.

Als inerte Reste eignen sich beispielsweise:
- C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, tert.-Amyl, n-Hexyl, iso-Hexyl, n-Heptyl, iso-Heptyl, n-Octyl und iso-Octyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
- C₃- bis C₈-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl.

Geeignete α,ω-Aminoalkohole der allgemeinen Formel I und deren α,ω-Alkandiole der allgemeinen Formel II sind z.B.:

| α,ω-Aminoalkohol I | α,ω-Alkandiol II |
|---|---|
| 1-Amino-propanol | Propandiol |
| 1-Amino-butanol | Butandiol |
| 1-Amino-pentanol | Pentandiol |
| 1-Amino-hexanol | Hexandiol |
| 1-Aminooctanol | Octandiol |
| Besonders bevorzugt | |
| 1-Aminobutanol | Butandiol-1,4 |
| 1-Aminopentanol | Pentandiol-1,5 |
| 1-Aminohexanol | Hexandiol-1,6 |

Die nach dem erfindungsgemäßen Verfahren herstellbaren Aminoalkohole der allgemeinen Formel I sind Zwischenprodukte für die Herstellung von Pharmaceutica (Lit: US 4151204) sowie potentiell für die Herstellung von Thixotropogenen, Farbstoffen und Korrosionsschutzmitteln.

### Beispiele

### Herstellung des Katalysators:

Ein Katalysator mit einem relativ geringen Schüttgewicht wird nach folgendem speziellen Verfahren hergestellt; die angestrebte Zusammensetzung entspricht z.B. 51,22 % Fe₂O₃ und 48,78 % Co₃O₄ (50 Gew.-% Fe zu 50 Gew.-% Co):
60 kg 20 %ige Sodalösung werden in einem Rührgefäß vorgelegt, auf etwa 70°C erhitzt und unter Rühren bei konstanter Temperatur ein Gemisch von 33 kg technischer Eisennitratlösung mit einem Gehalt von 10,86 % Fe₂O₃ und 25,72 kg Kobaltnitratlösung mit einem Gehalt von 16,48 % Co₃O₄ im Verlauf von 4 Stunden zudosiert, bis ein pH-Wert, mit der Glaselektrode gemessen, von 7,0 erreicht ist. Dann wird über einen Regelmechanismus, der den Zulauf steuert, unter Einhaltung des pH-Werts von 7,0 allmählich die restliche Menge Nitratlösung zugegeben. Der entstehende Niederschlag wird abfiltriert, mit vollentsalztem Wasser gewaschen und bei 120°C getrocknet.

Der Niederschlag enthält noch 0,47 % Na₂O bei einem Glühverlust von 23 %; er besteht röntgenographisch aus einem Gemisch von etwas CoCO₃ (mäßig gut kristallisiert) und sehr viel von einer mannasseitähnlichen Struktur (feinkristallisiert).

Der Niederschlag wird nun bei 350°C an der Luft zersetzt. Die Hauptmenge der chemisch gebundenen flüchtigen Bestandteile entweicht zwischen 145 und 200°C (Maximum bei 174°C) und zwischen 220 und 265°C (Maximum bei 253°C). Man erhält ein Pulver, das ein Schüttgewicht von 458 g/l und einen Glühverlust bei 900°C von 4,4 % aufweist. Es wird unter Zugabe von 2 % verdünnter Salpetersäure und ausreichend Wasser geknetet, stranggepreßt, getrocknet und bei 400°C getempert. Man erhält Extrudate, die ein Litergewicht von 900 g bei einer Porosität von 0,42 cm³/g und einer Schneidehärte von 2,5 kg aufweisen. Die Stränge lassen sich im Bereich von 265 bis 365°C (Maximum bei 336°C) in Wasserstoffatmosphäre bei schwach exothermer Reaktion vollständig reduzieren. Nach der Reduktion liegt röntgenographisch ein kubisch raumzentrierter Mischkristall zwischen Fe und Co vor.

Ein derart hergestellter Katalysator eignet sich nicht nur zur Aminierung von gesättigten Aminoalkoholen, sondern auch zur Hydrierung von Nitrilen und zur gleichzeitigen Hydrierung und Aminierung von Aminoalkenolen und Aminoalkinolen. Er ist also zur Hydrierung von C-N-Mehrfachbindungen, C-C-Doppel- und Dreifachbindungen in der Lage.

### Beispiel 1

Der Reaktor enhält 2,5 l des zuvor beschriebenen Katalysators mit 50 % Eisen und 50 % Kobalt. Er wurde im Verlauf von 2 Tagen mit Wasserstoff reduziert, wobei die Temperatur von 200 auf 300°C gesteigert wurde.

Die Reaktortemperatur wurde auf 230°C eingestellt und der Katalysator mit Ammoniak benetzt. Anschließend wurden je Stunde 1,7 l Pentandiol (95 %ige Ware) und 5 l Ammoniak (als Flüssigkeit gemessen) von oben nach unten durch den Reaktor geleitet. Dabei wurde durch Wasserstoff ein Druck von 200 bar aufrecht erhalten und stündlich 200 Normal-Liter Wasserstoff als Abgas abgezogen. In einem mehrwöchtigen Dauerversuch hatte der Reaktoraustrag im Mittel folgende Zusammensetzung (ammoniak- und wasserfrei gerechnet):

| | |
|---|---|
| Pentanolamin | 43 Gew.-% |
| Piperidin | 14 Gew.-% |
| Diaminopentan | 2 Gew.-% |
| nicht umgesetztes Pentandiol | 34 Gew.-% |
| Sonstige Nebenprodukte | 7 Gew.-% |

Der Anteil der Nebenprodukte entspricht etwa dem Anteil der Verunreinigungen im eingesetzten Pentandiol. Die Selektivität betrug 65 %.

### Beispiel 2

Analog Beispiel 1 wurde eine 50 %ige Lösung von Hexandiol in N-Methylmorpholin in einer Menge von 2,5 l Lösung je Stunde durch den Reaktor geleitet.

In einem mehrwöchigen Dauerversuch hatte der Reaktoraustrag (Ammoniak-, Wasser- und N-Methylmorpholin-frei gerechnet) folgende mittlere Zusammensetzung:

| | |
|---|---|
| Hexanolamin | 43 Gew.-% |
| Hexamethylenimin | 5 Gew.-% |
| Hexamethylendiamin | 13 Gew.-% |
| nicht umgesetztes Hexandiol | 36 Gew.-% |
| Sonstige Nebenprodukte | 3 Gew.-% |

Die Selektivität betrug 68 %.

### Beispiel 3a

Analog Beispiel 1 wurden je Stunde 0,75 l Diethylenglykol und 2,2 l Ammoniak bei 240°C durch den Reaktor geleitet und der Druck im Reaktor anstelle von Wasserstoff durch Stickstoff aufrecht erhalten.

Im Mittel hatte der Reaktoraustrag (Ammoniak- und Wasser-frei gerechnet) folgende Zusammensetzung:

| | |
|---|---|
| Aminodiglykol | 28 Gew.-% |
| Morpholin | 2 Gew.-% |
| nicht umgesetztes Diglykol | 62 Gew.-% |
| Nebenprodukte | 8 Gew.-% |

Die Selektivität betrug 74 %.

### Beispiel 3b

Analog Beispiel 3a wurde der Reaktorinnendruck mit Wasserstoff aufrechterhalten.

Im Mittel hatte der Reaktoraustrag (Ammoniak- und Wasser-frei gerechnet) folgende Zusammensetzung:

| | |
|---|---|
| Aminodiglykol | 44 Gew.-% |
| Morpholin | 18 Gew.-% |
| nicht umgesetztes Diglykol | 32 Gew.-% |
| Nebenprodukte | 6 Gew.-% |

Die Selektivität betrug 65 %.

### Vergleichsbeispiele mit anderen Katalysatoren

a) Analog Beispiel 1 wurde ein Katalysator mit folgender Zusammensetzung eingefüllt und unter den angegebenen Bedingungen reduziert:

| | |
|---|---|
| | 66 % CoO |
| | 20 % CuO |
| | 7 % Mn₃O₄ |
| | 4 % MoO₃ |
| | 3 % H₃PO₄ |
| Zulauf | 0,75 l/h Diethylenglykol |
| | 2,2 l/h NH₃ |
| Temperatur | 150, 170, 190°C |
| Druck | 250 bar H₂ |

Zusammensetzung des Austrags:

| | 150°C | 170°C | 190°C |
|---|---|---|---|
| Aminodiglykol | 13 | 17 | 20 Gew.-% |
| Morpholin | 7 | 10 | 34 Gew.-% |
| nicht umgesetztes Diglykol | 71 | 67 | 4 Gew.-% |
| Nebenprodukte | 9 | 6 | 42 Gew.-% |
| Selektivität | 45 | 51 | 20 Gew.-% |
| Umsatz | 29 | 33 | 96 Gew.-% |

b) Analog Beispiel 1 wurde ein Katalysator mit folgender Zusammensetzung eingefüllt und unter den angegebenen Bedingungen reduziert:

| | |
|---|---|
| | 46,5 % NiO |
| | 16,5 % CuO |
| | 30,0 % Al₂O₃ |
| | 1,4 % MoO₃ |
| Zulauf | 0,75 l/h Diethylenglykol |
| | 2,2 l/h NH₃ |
| Temperatur | 150 und 180°C |
| Druck | 200 bar H₂ |

Zusammensetzung des Austrags:

| | 170°C | 190°C |
|---|---|---|
| Aminodiglykol | 16 | 26 Gew.-% |
| Morpholin | 8 | 29 Gew.-% |
| n. u. Diglykol | 72 | 28 Gew.-% |
| Nebenprodukte | 4 | 18 Gew.-% |
| Selektivität | 57 | 20 % |
| Umsatz | 28 | 72 % |

c) Analog Beispiel 1 wurde ein Katalysator mit folgender Zusammensetzung eingefüllt und unter den angegebenen Bedingungen reduziert:

| | |
|---|---|
| | 4 % CuO |
| | 10 % CoO |
| | 10 % NiO |
| | auf Al₂O₃ |
| Zulauf | 0,75 l/h Hexandiol |
| | 2,2 l/h NH₃ |
| Temperatur | 160, 170, 180°C |
| Druck | 250 bar H₂ |

| | 160°C | 170°C | 180°C |
|---|---|---|---|
| Hexanolamin | 13 | 20 | 21 Gew.-% |
| Hexamethylenimin | 1 | 2 | 5 Gew.-% |
| Hexamethylendiamin | 2 | 4 | 6 Gew.-% |
| nicht umgesetztes Hexandiol | 84 | 70 | 62 Gew.-% |
| Sonstige | - | 4 | 6 Gew.-% |
| Selektivität | 79 | 66 | 55 Gew.-% |
| Umsatz | 16 | 30 | 38 Gew.-% |

d) Analog Beispiel 1 wurde ein Katalysator mit folgender Zusammensetzung eingefüllt und unter den angegebenen Bedingungen reduziert:

| | |
|---|---|
| | 23 % CoO |
| | 1 % Mn₃O₄ |
| | 0,5 % H₃PO₄ |
| | 0,2% Na₂O |
| | auf Al₂O₃ |
| Zulauf | 0,75 l/h Pentandiol |
| | 2,2 l/h NH₃ |
| Temperatur | 160, 170, 180°C |
| Druck | 250 bar H₂ |

| | 160°C | 170°C | 180°C |
|---|---|---|---|
| Pentanolamin | 11 | 14 | 16 Gew.-% |
| Piperidin | 4 | 5 | 7 Gew.-% |
| n. u. Pentandiol | 79 | 72 | 58 Gew.-% |
| Diaminopentan | - | - | - |
| Sonstige | 6 | 9 | 20 Gew.-% |
| Selektivität | 51 | 49 | 36 % |
| Umsatz | 21 | 28 | 42 % |

Die Vergleichsbeispiele a bis d zeigen qualitativ das gleiche Bild wie die Beispiele 1 bis 3, etwa in der Temperaturabhängigkeit der Selektivität. Quantitativ zeigen sie erhebliche Unterschiede: Bei gleicher Temperatur ist die Selektivität erheblich niedriger als bei Verwendung von Katalysatoren auf Eisenbasis. Den Vorteil der Zumischung von Kobalt zum Eisen zeigt das

### Beispiel 5

Analog Beispiel 1 wurden je Stunde 0,37 l Pentandiol und 1,2 l Ammoniak (flüssig) dem Reaktor zugeführt. Als Katalysator wurde ein praktisch nur aus Eisen bestehender Katalysator, der kein Kobalt enthielt, verwendet (analog EP-A-101 584); Katalysatorzusammensetzung vor der Reduktion:

| | |
|---|---|
| Eisen | 93,30 Gew.-% |
| Alkali (K + Na) | 0,03 Gew.-% |
| Mangan | 0,09 Gew.-% |
| Graphit | 2,40 Gew.-% |
| Sauerstoff | 4,80 Gew.-% |

Zusammensetzung des Austrags:

| | 240°C | 250°C |
|---|---|---|
| Pentanolamin | 32 | 34 % |
| Piperidin | 5 | 9 % |
| Pentandiol | 58 | 52 % |
| Diaminopentan | - | - |
| Sonstige | 5 | 5 % |
| Selektivität | 75 | 71 % |
| Umsatz | 42 | 48 % |

## Patentansprüche

1. Verfahren zur Herstellung von α,ω-Aminoalkoholen der allgemeinen Formel I
HO-CH₂-X-CH₂-NH₂ (I),
in der
X eine gegebenenfalls durch inerte Reste substituierte und/oder gegebenenfalls durch Sauerstoff oder Stickstoff unterbrochene C₁- bis C₂₀-Alkylenkette
bedeutet, durch Umsetzung von α,ω-Alkandiolen der allgemeinen Formel II
HO-CH₂-X-CH₂-OH (II),
in der das Zwischenglied X die oben genannten Bedeutungen hat, mit Ammoniak und einem Katalysator bei Temperaturen von 150 bis 300°C und Drücken von 50 bis 300 bar, dadurch gekennzeichnet, daß man Katalysatoren einsetzt, deren katalytisch aktive Masse zu 5 bis 100 Gew.-% aus Eisen besteht.

2. Verfahren zur Herstellung von α,ω-Aminoalkoholen nach Anspruch 1, dadurch gekennzeichnet, daß man Katalysatoren einsetzt, deren katalytisch aktive Masse zu 5 bis 100 Gew.-% aus Eisen und zu 0 bis 95 Gew.-% aus Kobalt besteht.

3. Verfahren zur Herstellung von α,ω-Aminoalkoholen nach Anspruch 1, dadurch gekennzeichnet, daß man die Reaktion in Anwesenheit von Wasserstoff und/oder Stickstoff durchführt.

## Claims

1. A process for the preparation of α,ω-amino alcohols of the general formula I
HO-CH₂-X-CH₂-NH₂ (I),
where X is a C₁- to C₂₀-alkylene chain which is unsubstituted or substituted by inert radicals and/or may be interrupted by oxygen or nitrogen,
by reacting α,ω-alkanediols of the general formula II
HO-CH₂-X-CH₂-OH (II),
where the intermediate member X has the abovementioned meanings, with ammonia and a catalyst at from 150 to 300°C and under from 50 to 300 bar, wherein catalysts whose catalytically active mass comprises from 5 to 100 % by weight iron are used.

2. A process for preparing α,ω-amino alcohols as claimed in claim 1, wherein catalysts whose catalytically active mass comprises from 5 to 100 % by weight iron and from 0 to 95 % by weight cobalt are used.

3. A process for preparing α,ω-amino alcohols as claimed in claim 1, wherein the reaction is carried out in the presence of hydrogen and/or nitrogen.

## Revendications

1. Procédé de préparation d'α,ω-aminoalcools de la formule générale I :
HO-CH₂-X-CH₂-NH₂ (I)
dans laquelle
X représente une chaîne alkylène en C₁ à C₂₀ éventuellement substituée par des radicaux inertes et/ou éventuellement interrompue par de l'oxygène ou de l'azote,
par la réaction d'α,ω-alcanediols de la formule générale II :
HO-CH₂-X-CH₂-OH (II)
dans laquelle
le groupement intermédiaire X possède les significations qui lui ont été attribuées ci-dessus, avec de l'ammoniac et un catalyseur, à des températures de 150 à 300°C et sous des pressions de 50 à 300 bars, caractérisé en ce que l'on utilise des catalyseurs dont la masse catalytiquement active se compose pour 5 à 100% en poids de fer.

2. Procédé de préparation d'α,ω-aminoalcools suivant la revendication 1, caractérisé en ce que l'on utilise des catalyseurs dont la masse catalytiquement active se compose jusqu'à 5 à 100% en poids de fer et jusqu'à 0 à 95% en poids de cobalt.

3. Procédé de préparation d'α,ω-aminoalcools suivant la revendication 1, caractérisé en ce que l'on entreprend la réaction en présence d'hydrogène et/ou d'azote.
